# Europäisches Patentamt

**European Patent Office**

**Office européen des brevets**

(11) Veröffentlichungsnummer: **0 048 978**
A2

(12) **EUROPÄISCHE PATENTANMELDUNG**

(21) Anmeldenummer: **81107647.0**

(22) Anmeldetag: **25.09.81**

(51) Int. Cl.³: **B 66 F 7/14**

(30) Priorität: **30.09.80 DE 3036940**

(43) Veröffentlichungstag der Anmeldung: **07.04.82**
Patentblatt 82/14

(84) Benannte Vertragsstaaten: **DE FR GB SE**

(71) Anmelder: **Siemens-Elema AB, Röntgenvägen 2, S-171 95 Solna 1 (SE)**

(84) Benannte Vertragsstaaten: **SE**

(71) Anmelder: **SIEMENS AKTIENGESELLSCHAFT Berlin und München, Postfach 22 02 61, D-8000 München 22 (DE)**

(84) Benannte Vertragsstaaten: **DE FR GB**

(72) Erfinder: **Stödberg, Lars B., Klevbergsvägen 81, S-17900 Stenhamra (SE)**

(54) Schrauben-Mutter-Transmission zum vertikalen Verschieben einer Last (Fallsicherung).

(57) Zum vertikalen Verschieben einer Last mit einer Schrauben-Mutter-Transmission ist die Last fest mit einer Arbeitsmutter verbunden. Um auch bei abgeschertem Gewinde ein Durchsacken der Last zu verhindern, ist erfindungsgemäss auf der Schraube (3) unterhalb der Arbeitsmutter (5) in einem bestimmten Abstand zu dieser eine in einem Ring (61) eines Wälzlagers (6) angeordnete Hilfsmutter (7) vorgesehen. Diese Hilfsmutter ist unter normalen Umständen drehgesichert und läuft unbelastet mit. Fällt die Arbeitsmutter (5) mit der Last durch, so kommt sie direkt oder indirekt auf den anderen Ring (62) des Wälzlagers (6) zu liegen. Die Hilfsmutter (7) übernimmt die Last und kann sich gleichzeitig über den Innenring zusammen mit der Schraube (3) drehen, so dass eine axiale Bewegung unmittelbar aufhört.

0048978

SIEMENS AKTIENGESELLSCHAFT          Unser Zeichen
Berlin und München                  VPA 80 P 5959  E

Schrauben-Mutter-Transmission zum vertikalen Verschieben
einer Last

Die Erfindung betrifft eine Schrauben-Mutter-Transmission zum überwiegend vertikalen Verschieben einer mit einer Arbeitsmutter fest verbundenen Last. Derartige Transmissionen sind z. B. bei einem Patienten-Auflagetisch für Untersuchungen und Behandlungen mit Röntgenstrahlen bekannt. Dabei ist die Schraube häufig in einem Drehlager an der Decke befestigt und von einem Rohr umschlossen. Der Patienten-Auflagetisch hängt über ein zweites Rohr, das in dem ersten teleskopartig verschiebbar ist, an der Arbeitsmutter. Die beiden Rohre können auch einen eckigen Innenquerschnitt aufweisen und somit ein Verdrehen gegeneinander verhindern.

Unter Umständen kann es insbesondere nach längerem Gebrauch, durch unsachgemäße Behandlung oder durch zeitweise Überbelastung zu einer Beschädigung des Mutterngewindes kommen, das letztendlich zu einem Abscheren des Gewindes und damit zu einem plötzlichen Durchsacken der Last, z. B. eines Patienten-Auflagetisches mit einem Patienten, führt.

Der vorliegenden Erfindung liegt die Aufgabe zugrunde, ohne Beeinträchtigung des normalen Hebens und Senkens der Last für eine Schrauben-Mutter-Transmission der eingangs genannten Art eine derartige Fallsicherung zu schaffen, daß auch bei abgeschertem oder sonst die Last nicht mehr tragendem Gewinde diese Last nahezu unverändert in der eingestellten Höhe verharrt und die mögliche Rotation der Schraube völlig unbehindert bleibt.

Gdl 5 Lo / 23.9.1980

Diese Aufgabe wird erfindungsgemäß dadurch gelöst, daß auf der Schraube unterhalb der Arbeitsmutter in einem bestimmten Abstand zu dieser eine in einem Ring eines Wälzlagers angeordnete Hilfsmutter vorgesehen ist, die gekoppelt über die Arbeitsmutter ab einem Minimalabstand zwischen den Muttern drehgesichert ist, und daß beim Unterschreiten dieses Minimalabstandes die Arbeitsmutter direkt oder indirekt auf den anderen Ring des Wälzlagers zu liegen kommt.

Unter normalen Arbeitsbedingungen befindet sich die Hilfsmutter in einem bestimmten Abstand unterhalb der Arbeitsmutter und ist unbelastet. Wird die Schraube gedreht, so heben oder senken sich die beiden Muttern parallel, weil die Hilfsmutter über die Arbeitsmutter gekoppelt drehgesichert ist. Ist das Gewinde der Arbeitsmutter derart beschädigt, daß diese durchfällt, landet sie direkt oder indirekt auf dem anderen Ring des Wälzlagers und der Fall wird unmittelbar gestoppt. Dabei löst sich gleichzeitig die Drehsicherung und die Hilfsmutter dreht sich zusammen mit der Schraube, so daß die Hilfsmutter in ihrer bisherigen Höhenlage verbleibt. Da der Abstand zwischen den beiden Muttern sehr klein gehalten werden kann, bleibt die Last praktisch unverändert in ihrer eingestellten Lage. Für einen Patienten auf einem Patienten-Auflagetisch z. B. wäre ein solches "Durchfallen" nur noch als ein kaum merkbarer Stoß spürbar. Eine Gefährdung des Patienten oder des sich in der Nähe befindenden Bedienungs- oder Behandlungspersonals wird damit sicher ausgeschlossen.

Da sich die Schraube ungestört weiterdrehen kann, wird auch eine weitere Beschädigung der Transmission oder z. B. eine Überbelastung eines die Schraube drehenden Motors vermieden.

Weiterhin kann die Schrauben-Mutter-Transmission so konzipiert sein, daß das Gewinde der Mutter bewußt stets
stärker beansprucht und damit auch stärker abgenutzt
wird als das Gewinde der Schraube. Damit ist sichergestellt, daß in einem Schadensfall immer die Mutter zuerst
versagt und die erfindungsgemäße Fallsicherung wirksam
bleibt.

Als Wälzlager kann ein Axiallager vorgesehen sein. Die
Hilfsmutter ist dann in dem Ring angeordnet, der sich auf
der von der Arbeitsmutter abgewandten Seite des Wälzlagers befindet. Beim Durchfallen kann die Arbeitsmutter
direkt auf den ihr zugewandten Ring dieses Lagers aufstoßen. Ebenso vorteilhaft kann als Wälzlager ein Radiallager vorgesehen sein. Die Hilfsmutter ist dann im Innenring dieses Lagers angeordnet. Die Arbeitsmutter darf im
Schadensfall nur auf dem Außenring aufliegen. Dazu kann
der Außenring etwas breiter als der Innenring sein, oder
die Arbeitsmutter kann in dem entsprechenden Bereich an
ihrer Unterseite einen Vorsprung aufweisen. In Weiterbildung der Erfindung ergibt sich eine besonders einfache
Lösung durch eine Distanzscheibe, die sich zwischen der
Arbeitsmutter und dem Außenring des Wälzlagers befindet.

Eine konstruktiv besonders einfache Drehsicherung für die
Hilfsmutter ergibt sich durch eine Senkung, die die
Hilfsmutter an ihrer Unterseite aufweist, und durch einen
mit der Arbeitsmutter direkt oder indirekt gekoppelten
Mitnehmerzapfen, der in diese Senkung eingreift. Sackt
die Arbeitsmutter ab, so verkleinert sich der Abstand
zwischen beiden Muttern und der Mitnehmerzapfen gleitet
aus der Senkung nach unten heraus. Die Drehsicherung ist
damit beseitigt.

Die zu hebende Last kann nahezu beliebig mit der Arbeitsmutter verbunden sein. Die einzige Bedingung ist der

- 4 -     VPA 80 P 5959 E

starre Abstand zwischen beiden. Dafür können z. B. Stangen vorgesehen sein. In einer vorteilhaften Weiterbildung der Erfindung ist als Verbindung ein Rohr vorgesehen, das die Arbeitsmutter, das Wälzlager und gegebenenfalls die Distanzscheibe umschließt. Die Arbeitsmutter ist dabei fest mit dem Rohr verbunden, während die übrigen Teile in ihm gleiten können. Bei dieser Anordnung läßt sich die Drehsicherung besonders einfach dadurch erreichen, daß der Mitnehmerzapfen in dem geforderten Abstand von der Arbeitsmutter an dem Rohr befestigt ist.

Das Rohr kann von einem Außenrohr derart umschlossen sein, daß beide Rohre zusammen in bekannter Weise eine Teleskopsäule bilden. Das Außenrohr kann z. B. an einer Decke befestigt sein. Insgesamt ergibt sich damit eine gegen Verschmutzung abgeschirmte Transmission, die äußerlich leicht zu reinigen ist, was insbesondere für den Einsatz im Krankenwesen notwendig ist.

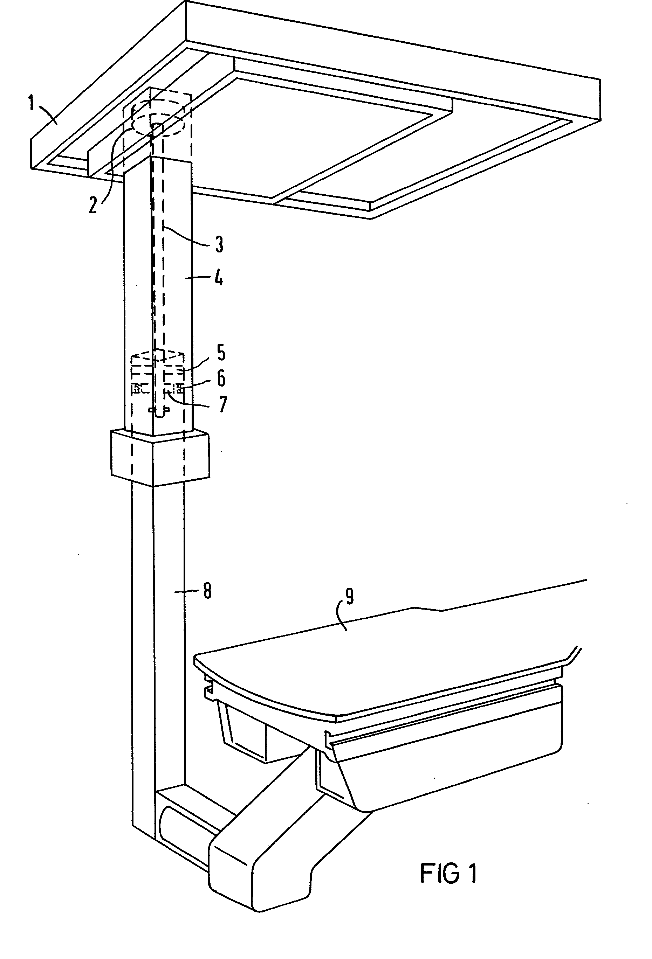

Die Erfindung wird im folgenden anhand von in drei Figuren dargestellten Ausführungsbeispielen näher beschrieben und erläutert.
Dabei zeigt Fig. 1 in perspektivischer Darstellung einen hängenden Patienten-Auflagetisch mit der erfindungsgemäßen Fallsicherung.
Fig. 2 zeigt im Schnitt einen vergrößerten Ausschnitt aus Fig. 1 mit der Arbeits- und der Hilfsmutter.
Fig. 3 zeigt einen entsprechenden Ausschnitt wie Fig. 2 mit einem anderen Wälzlager.

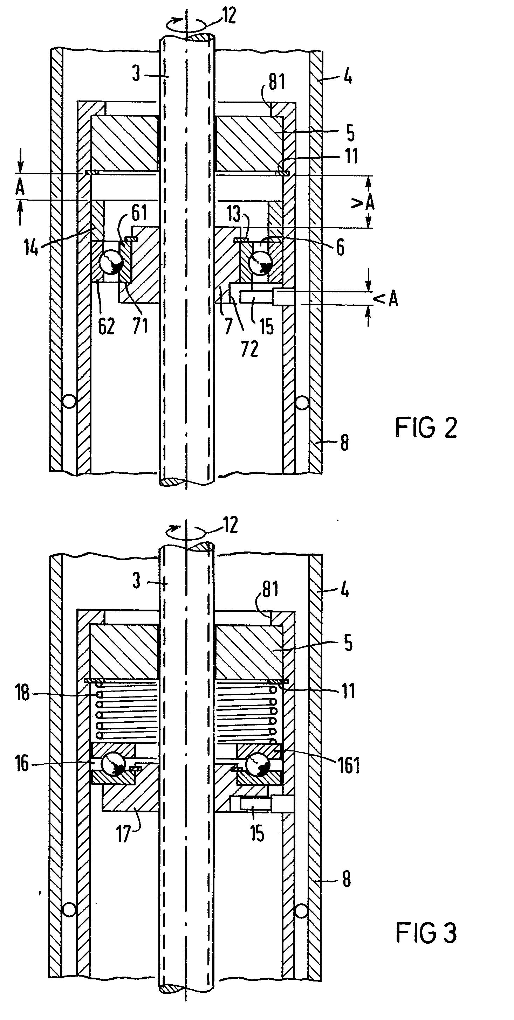

Die Figur 1 zeigt eine Platte 1, die an einer nicht dargestellten Decke befestigt ist. In dieser Platte sitzt ein Drehlager 2 mit einer senkrecht nach unten in den Raum hineinragenden Schraube 3. Die Schraube 3 kann z. B. über einen ebenfalls nicht dargestellten Motor in bekannter Weise gedreht werden. Das Drehlager 2 und die Schraube 3 sind von einem Außenrohr 4 mit quadratischem Querschnitt umgeben, das auch in der Platte 1 verankert ist. Auf der Schraube 3 sitzt die Arbeitsmutter 5 und die in einem Wälzlager 6 gehaltene Hilfsmutter 7, auf deren Funktion anhand der Figuren 2 und 3 näher eingegangen wird. Die beiden Muttern und das Wälzlager sind lediglich gestrichelt angedeutet.

Die Arbeitsmutter 5 ist fest mit dem oberen Ende eines weiteren Rohres 8 quadratischen Querschnitts verbunden, das teleskopartig in dem Außenrohr verschiebbar ist. Am unteren Ende des Rohres 8 ist über weitere Verbindungsstücke, auf die hier nicht weiter eingegangen wird, der Patienten-Auflagetisch 9 als Last angeflanscht. Dieser Tisch 9 kann durch Drehen der Schraube 3 in seiner Höhe verstellt werden.

Fig. 2 zeigt einen vergrößerten Ausschnitt aus Fig. 1. Gleiche Teile sind mit gleichen Bezugszeichen versehen. Die Arbeitsmutter 5 ist mit dem Rohr 8 drehsicher und fest verbunden. Das Rohr 8 weist dazu an seinem oberen Ende einen Absatz 81 auf, gegen den die Arbeitsmutter 5 mittels eines Spannringes 11 gepreßt wird. Wenn die Schraube 3 um ihre Achse gedreht wird, wie es durch den Pfeil 12 angedeutet ist, wird die Mutter 5 und mit ihr das Rohr 8, wenn es am Mitdrehen gehindert wird, axial entlang der Schraube 3 verschoben. Unterhalb der Arbeitsmutter 5 befindet sich eine Hilfsmutter 7, die in einem radialen Wälzlager 6 gehalten ist. Dazu weist die Hilfsmutter 7 einen Absatz 71 auf, gegen den der Innenring 61 des Wälzlagers 6 mittels eines Spannringes 13 gepreßt wird. Der Außenring 62 des Wälzlagers 6 ist in dem Rohr 8 frei bewegbar. Die Hilfsmutter 7 weist weiterhin an ihrer Unterseite eine Senkung 72 auf. Zwischen dem Außenring 62 des Wälzlagers 6 und der Arbeitsmutter 5 befindet sich eine im Rohr 8 verschiebbare Distanzscheibe 14. Der freie Abstand zwischen dieser Distanzscheibe 14 und der Arbeitsmutter 5 ist mit A bezeichnet. Im normalen Arbeitszustand,
d. h. bei einwandfrei ausgebildetem Gewinde der Arbeitsmutter, trägt diese die ganze Last. Die Hilfsmutter läuft
unbelastet mit. Der Abstand zwischen beiden Muttern ist
größer als A. In der Höhe der Senkung 72 ist in das Rohr 8
ein Mitnehmerzapfen 15 fest eingesetzt, der in die Senkung 72 eingreift. Der Abstand der Oberkante dieses Zapfens 15 über der Unterkante der Hilfsmutter ist kleiner
als A. Durch den Mitnehmerzapfen 15 wird die Hilfsmutter
am Mitdrehen gehindert, wenn die Schraube 3 sich dreht.
Beide Muttern werden dadurch mit konstantem Abstand entlang der Schraube nach oben oder unten verschoben.

Fällt bei einer Beschädigung des Gewindes der Arbeitsmutter 5 diese durch, so kommt sie über die Distanzscheibe 14
auf dem Außenring 62 des Wälzlagers 6 zu liegen. Der Abstand zwischen beiden Muttern verringert sich um A, wodurch der Mitnehmerzapfen 15 nach unten aus der Senkung 72
herausgleitet. Die Hilfsmutter 7 trägt nunmehr die Last.

Wird in einem solchen Schadensfall gerade die Schraube 3
gedreht, so rotiert die Hilfsmutter 7 über den Innenring 61 des Wälzlagers 6 zusammen mit der Schraube 3. Die
axiale Bewegung der Last wird dadurch automatisch gestoppt. Gefahr für die Last, insbesondere für einen Patienten, besteht durch diese Fallsicherung nicht mehr. Daß
sich trotz rotierender Schraube 3 die Last nicht in der
Höhe verschiebt, ist gleichzeitig ein Hinweis für das
Bedienungspersonal, daß ein Defekt vorliegt. Dieser Hinweis kann im Bedarfsfall leicht über Kontakte oder Meßfühler in ein optisches oder akustisches Signal umgewandelt werden.

Fig. 3 zeigt eine abgewandelte Ausführungsform, bei der
anstelle des in Fig. 2 dargestellten Radiallagers 6 ein Axiallager 16 verwendet ist, zwischen dem und der Arbeitsmutter 5 eine Feder 18 angeordnet ist, die den oberen
Ring 161 des Axiallagers 16 am Platz hält. Es kann auch
noch eine Distanzscheibe wie bei dem Ausführungsbeispiel
gemäß Fig. 2 vorgesehen sein. Die Hilfsmutter 17 ist an
das andere Wälzlager 16 angepaßt. Ansonsten ist die Anordnung unverändert geblieben. Auch an der Wirkungsweise
hat sich nichts geändert.

3 Figuren
7 Ansprüche

0048978

- 8 -    VPA 80 P 5959 E

<u>Patentansprüche</u>

1. Schrauben-Mutter-Transmission zum überwiegend vertikalen Verschieben einer mit einer Arbeitsmutter fest verbundenen Last, **d a d u r c h   g e k e n n z e i c h - n e t ,** daß auf der Schraube (3) unterhalb der Arbeitsmutter (5) in einem bestimmten Abstand zu dieser eine in einem Ring (61) eines Wälzlagers (6, 16) angeordnete Hilfsmutter (7, 17) vorgesehen ist, die gekoppelt über die Arbeitsmutter (5) ab einem Minimalabstand zwischen den Muttern (5; 7, 17) drehgesichert ist, und daß beim Unterschreiten dieses Minimalabstandes die Arbeitsmutter (5) direkt oder indirekt auf den anderen Ring (62) des Wälzlagers (6, 16) zu liegen kommt.

2. Schrauben-Mutter-Transmission nach Anspruch 1, **d a - d u r c h   g e k e n n z e i c h n e t ,** daß das Wälzlager ein Axiallager (16) ist.

3. Schrauben-Mutter-Transmission nach Anspruch 2, **d a - d u r c h   g e k e n n z e i c h n e t ,** daß zwischen der Arbeitsmutter (5) und dem der Arbeitsmutter (5) zugewandten Ring (161) des Axiallagers (16) eine Feder (18) angebracht ist.

4. Schrauben-Mutter-Transmission nach Anspruch 1, **d a - d u r c h   g e k e n n z e i c h n e t ,** daß das Wälzlager ein Radiallager (6) ist.

5. Schrauben-Mutter-Transmission nach Anspruch 4, **d a - d u r c h   g e k e n n z e i c h n e t ,** daß sich zwischen der Arbeitsmutter (5) und dem Außenring (62) des Wälzlagers (6) eine Distanzscheibe (14) befindet.

6. Schrauben-Mutter-Transmission nach einem der Ansprüche 1 bis 5, d a d u r c h  g e k e n n z e i c h - n e t , daß die Hilfsmutter (7, 17) an der Unterseite eine Senkung (72) aufweist, in die zur Drehsicherung ein Mitnehmerzapfen (15) eingreift.

7. Schrauben-Mutter-Transmission nach einem der Ansprüche 1 bis 6, d a d u r c h  g e k e n n z e i c h - n e t , daß die Last (9) mit der Arbeitsmutter (5) über ein Rohr (8) verbunden ist, das diese Mutter (5), das Wälzlager (6, 16) und gegebenenfalls die Distanzscheibe (14) umschließt.

0048978

80 P 5959

FIG 1

0048978
80 P 5959

FIG 2

FIG 3